# EUROPEAN PATENT SPECIFICATION

(11) **EP 3 645 034 B1**
(45) Date of publication and mention of the grant of the patent: **30.08.2023**
(21) Application number: 18736860.0
(22) Date of filing: 29.06.2018
(51) Int. Cl.: A61K 38/48, C12N 9/64

(54) **21-DAY DOSING REGIMEN FOR FUSION PROTEINS COMPRISING FACTOR IX AND HUMAN ALBUMIN FOR PROPHYLACTIC TREATMENT OF HEMOPHILIA AND METHODS THEREOF**
21-TAGE-DOSIERSCHEMA FÜR FUSIONSPROTEINE MIT FAKTOR IX UND MENSCHLICHEM ALBUMIN ZUR PROPHYLAKTISCHEN BEHANDLUNG VON HÄMOPHILIE UND VERFAHREN DAFÜR
SCHÉMA POSOLOGIQUE SUR 21 JOURS POUR PROTÉINES DE FUSION COMPRENANT UN FACTEUR IX ET UNE ALBUMINE HUMAINE POUR LE TRAITEMENT PROPHYLACTIQUE DE L'HÉMOPHILIE ET MÉTHODES ASSOCIÉES

(30) Priority: 29.06.2017 US 201762526377 P
(43) Date of publication of application: 06.05.2020
(73) Proprietor: CSL Behring Lengnau AG, 2543 Lengnau BE (CH)
(72) Inventor: LI, Yanyan, Furlong, PA 18925 (US); BENSEN-KENNEDY, Debra, Phoenixville, PA 19460 (US); JACOBS, Iris, King of Prussia, PA 19406 (US); VOIGT, Christine, Devon, PA 19333 (US)
(74) Representative: Pohlman, Sandra M.
(86) International application number: PCT/EP2018/067523
(87) International publication number: WO 2019/002532

(56) References cited:
- EP-A1- 3 086 804
- WO-A1-2014/052490
- E. SANTAGOSTINO ET AL: "Safety and pharmacokinetics of a novel recombinant fusion protein linking coagulation factor IX with albumin (rIX-FP) in hemophilia B patients", BLOOD, vol. 120, no. 12, 20 September 2012 (2012-09-20), pages 2405-2411, XP055355107, US ISSN: 0006-4971, DOI: 10.1182/blood-2012-05-429688
- MARTINOWITZ URI ET AL: "Phase I/II, open-label, multicenter, safety, efficacy and PK study of a recombinant coagulation factor IX albumin fusion protein (rIX-FP) in subjects with hemophilia B", THROMBOSIS RESEARCH, vol. 131, 1 January 2013 (2013-01-01), XP028525643, ISSN: 0049-3848, DOI: 10.1016/S0049-3848(13)70152-X
- SANTAGOSTINO ELENA: "PROLONG-9FPclinical development program - phase I results of recombinant fusion protein linking coagulation factor IX with recombinant albumin (rIX-FP)", THROMBOSIS RESEARCH, vol. 131, 1 January 2013 (2013-01-01), XP028525644, ISSN: 0049-3848, DOI: 10.1016/S0049-3848(13)70151-8
- SCHULTE ET AL: "Half-life extension through albumin fusion technologies", THROMBOSIS RESEARCH, TARRYTOWN, NY, US, vol. 124, 1 December 2009 (2009-12-01), pages S6-S8, XP026812470, ISSN: 0049-3848 [retrieved on 2009-12-01]

## Description

### Field of the Invention

The present invention relates to prophylactic dosing regimens with about 90-110 IU/kg long-acting factor IX (human FIX-human albumin) in dosing intervals of three weeks (21 days).

### Background of the invention

Hemophilia B is an X-linked recessive inherited bleeding disorder resulting from a deficiency of coagulation factor IX (FIX), a coagulation factor central to the process of blood coagulation. Signs and symptoms of hemophilia B are variable, depending on the severity of FIX deficiency and the location of bleeding. Most often, bleeding is characterized by spontaneous or trauma-induced hemorrhage into joints, muscles and soft tissues. Recurrent bleeding in the same location may lead to permanent injury of the joint. Rare but life-threatening bleeding may also occur in the central nervous system, throat, or gastrointestinal tract.

The goal of therapy for hemophilia B is to treat or prevent hemorrhage, thereby reducing disabling joint and tissue damage, and improving quality of life (QoL). Replacement therapy with FIX provides a temporary correction of the factor deficiency and reduces bleeding tendencies. Currently, plasma-derived and recombinant FIX products are used for the prophylactic and on-demand treatment of hemophilia B. However, plasma-derived products are associated with risks related to transmission of infectious viruses such as human immunodeficiency virus, hepatitis B virus, and hepatitis C virus. Both plasma-derived and recombinant FIX products have a short half-life and, therefore, require dosing 2 to 3 times a week in order to achieve a significant reduction of bleeding episodes. In addition, repeat dosing may be required to control bleeding episodes with a relatively short bleeding-free period following administration. The need for frequent intravenous (IV) injections of either plasma-derived or recombinant FIX products carries significant burden for patients and the physicians treating their disorder. Such a regimen in younger children often (but not always), requires the insertion of a venous access device that must be kept extremely clean to avoid infectious complications and prevent the development of clots in the line. The risk and morbidity associated with such devices may prevent some very young children with hemophilia from receiving adequate care.

A pegylated form of FIX has been generated which shows 5 to 7 times prolonged half-life of FIX in minipigs and dogs (∅stergaard, H, et al., 2011. Blood. 118(8):2333-2341). Similar results were observed in patients (Negrier C et al. Blood. 2011 Sep 8;118(10):2695-701). A fusion protein comprising recombinant FIX and albumin (rIX-FP) has demonstrated in rats, rabbits, and FIX-deficient mice that it has improved PK parameters (i.e., increased recovery, terminal half-life and area under the concentration-time curve [AUC]) compared with published results of a currently marketed recombinant FIX product (e.g., rFIX)) (Metzner HJ, et al., 2009. Thromb Haemost. 102:634-644). Studies in humans showed that weekly prophylaxis of rIX-FP decreased the consumption of FIX compared to the previous FIX product with fewer infusions (Martinowitz et al., ISTH, Amsterdam, The Netherlands, June 29-July 4, 2013 abstract).

Another example of such a fusion protein comprising FIX is rFIX-Fc. In WO 2012/006624, therapeutic chimeric polypeptides comprising FIX are described which can be fused to FcRn Binding Partners such as Fc or albumin. rFIX-Fc showed an increased half-life in vivo. This document suggests administering rFIXFc at 20 IU/kg weekly, 40 IU/kg every 10 days or 120 IU/kg every two weeks for prophylactic therapy. Luk et al. (2011. Haemophilia. 17:352-380) also describes that rFIX-Fc showed an increase in half-life, as well as in other PK parameters, compared to BeneFIX^{®} (i.e., rFIX). The phase 3 study for rFIX-Fc was recently published which shows that when administered at a weekly interval starting at 50 IU/kg or at 100 IU/kg starting at a 1 0-day dosing interval, there was low annualized bleeding rates in patients with hemophilia B (Powell JS, et al., Dec 12, 2013. NEJM. 369:2313-23).

WO2015/095925 describes fusion proteins comprising Factor IX for prophylactic treatment of hemophilia at various doses (25-250 lU/kg) and dosing intervals ranging from daily to once a month. ABR data is provided for weekly prophylaxis (25-35 IU/kg), and FIX activity levels are reported for five patients with hemophilia over 21 days who received 100 IU/kg. The regimen showed a FIX activity of at least 4.1% at day 21-post 100 IU/kg rIX-FP injection. PK results were simulated for a 100 IU/kg 21-day dosing interval. Zhang et al. (2016), Journal of Thrombosis and Haemostasis. 14:2132-2140) describes the prolonged half-life of rIX-FP and its population PK model supports prolonged dosing of rIX-FP with intervals of up to 2 weeks.

The present invention provides prophylactic dosing regimens with long-acting factor IX which is a fusion protein comprising a human Factor IX (FIX) and human albumin wherein the FIX is connected to the N-terminus of human albumin via a peptide linker cleavable by proteases involved in coagulation or activated by coagulation enzymes, and wherein the fusion protein is to be administered intravenously to the subject at a dose of about 90-110 IU/kg for a dosing interval of about 21 days.

The albumin increases the half-life of the human FIX, but without a cleavable linker, the FIX has in general reduced or low activity. The cleavable linker allows the polypeptide to be liberated from any activity-compromising steric hindrance caused by the albumin, thereby allowing the generation of fusion proteins, which retain a high molar specific activity of the FIX. Such fusion proteins exhibit improved half-life and molar specific activities that are increased in comparison to their non-cleavable counterparts. When cleavage occurs by proteins involved in coagulation, this allows the non-activated FIX to have an increased half-life until a bleeding event occurs, and simultaneous activation of FIX and cleavage from albumin. The activated FIX, which is liberated from its fusion to the albumin, has both high activity and is rapidly cleared from the blood due to the loss of albumin.

The prior art describes a fusion protein comprising FIX and albumin with a non-cleavable linker. For example, WO2001/79271 describes fusion polypeptides of a multitude of different therapeutic polypeptides which, when fused to human serum albumin, are predicted to have an increased functional half-life in vivo and extended shelf-life. Among the list of therapeutic polypeptides mentioned as Examples is Factor IX. Also described are fusions of FIX in which there is a peptide linker between albumin and FIX, but the linker is not specified to be cleavable. Sheffield et al. ((2004), Br. J. Haematol. 126: 565-573) expressed a murine Factor IX albumin fusion protein composed of murine FIX, a linker of 8 amino acids (GPG₄TM), murine albumin and a peptide tag of 22 amino acids, and also a human Factor IX albumin fusion protein composed of human Factor IX, a linker of 7 amino acids (G₆V) and human albumin. Sheffield does not use or suggest using a cleavable linker between FIX and albumin. These fusion proteins allow FIX to be activated but it has low activity due to the presence of albumin.

Once a coagulation factor is activated during coagulation either by proteolytic cleavage of the zymogen (like FIX) or by contact of an already proteolytically "pre"-activated factor to a second polypeptide (like FVlla binding to Tissue Factor), it is no longer desirable to maintain the long half-life of the now activated coagulation factor, as this might lead to thrombotic complications and should be even more relevant if the activated factor would have an increased half-life. It is therefore the objective of the present invention to provide long-lived human FIX suitable for prophylactic therapy with treatment periods of 21 days.

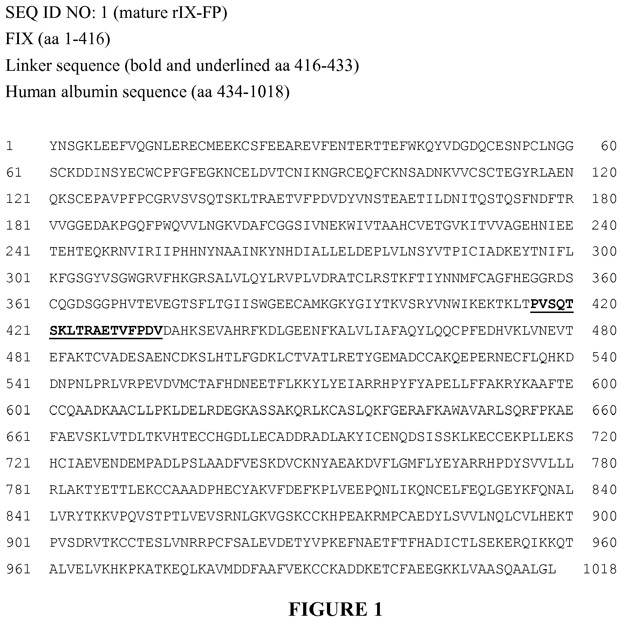

rIX-FP (recombinant human FIX-human albumin fusion protein) having the sequence set forth in SEQ ID NO: 1 (see Figure 1), showed improved PK parameters. Specifically, it has prolonged circulation in plasma as shown by the 5.3-fold longer half-life (t_{1/2}), the 7-fold reduced CL, and the 7-fold greater AUC compared to rFIX (e.g., Benefix^{®}) (Santagostino E, et al. Blood. 2012 Sep 20;120(12):2405-11). This surprisingly allows for prophylactic treatment of hemophilia with dosing intervals that are significantly longer than demonstrated by the prior art for FIX fusion proteins.

### Summary of the invention

The invention relates to a fusion protein comprising
a) a human Factor IX (FIX) portion, and
b) human albumin
for use in a method of preventing bleeding in a human suffering from hemophilia B in a prophylactic dosing regimen, wherein the FIX is connected to the N-terminus of human albumin via a peptide linker cleavable by proteases involved in coagulation or activated by coagulation enzymes, and wherein the fusion protein is to be administered to the subject at a dose of about 90-110 lU/kg for a dosing interval of once every 21 days. The dose is administered intravenously. In a highly preferred embodiment, the dose is about 100 IU/kg.

In any one of these embodiments, the dosing interval is once every 21 days.

In some embodiments, the said prophylactic dosing regimen of the long acting human FIX may result in an annualized bleeding rate of less than 20 per year, less than 15 per year, less than 10 per year, less than 9 per year, less than 8 per year, less than 7 per year, less than 6 per year, less than 5 per year, less than 4 per year, or less than 3 per year.

In a preferred embodiment, the said prophylactic dosing regimen of the long acting human FIX results in an annualized bleeding rate of less than 20 per year, most preferably less than 3 per year.

In some embodiments, the said prophylactic dosing regimen of the human long acting FIX may result in an annualized spontaneous bleeding rate of less than 15 per year, less than 12 per year, less than 10 per year, less than 9 per year, less than 8 per year, less than 7 per year, less than 6 per year, less than 5 per year, less than 4 per year, less than 3 per year, or less than 2 per year.

In a highly preferred embodiment, the said prophylactic dosing regimen of the long acting human FIX results in an annualized spontaneous bleeding rate of less than 15 per year, most preferably less than 2 per year.

For any of the embodiments of the invention, the plasma level of the FIX is maintained at a trough of at least about 1 % above baseline for the entire dosing interval. Preferably, the plasma level of the FIX is maintained at a trough of at least about 2-4% above baseline for the entire dosing interval, and more preferably at least about 2, 3, or 4% above baseline for the entire dosing interval. In other more preferred embodiments the plasma level of the FIX is maintained between 5 and 15% above baseline for the entire dosing interval.

The said fusion protein has a linker that is cleavable by the protease that activates the coagulation factor, thereby ensuring that the cleavage of the linker is linked to the activation of the coagulation factor at a site at which coagulation occurs. Other preferred fusion proteins, according to the invention, are those wherein the linker is cleavable by the coagulation factor which is part of the fusion protein once it is activated, thus also ensuring that cleavage of the fusion protein is connected with a coagulatory event. Other preferred fusion proteins according to the invention are those, wherein the linker is cleavable by a protease, which itself is activated directly or indirectly by the activity of the coagulation factor which is part of the fusion protein, thus also ensuring that cleavage of the fusion protein is connected with a coagulatory event.

In a preferred embodiment, the linker is cleavable by FIXa and/or by FVlla/Tissue Factor (TF).

In a particularly preferred embodiment, the linker comprises a sequence selected from SEQ ID NO: 2 and SEQ ID NO: 3:
SEQ ID NO: 2 
Pro Val Ser Gln Thr Ser Lys Leu Thr Arg Ala Glu Thr Val Phe Pro Asp Val
1               5                   10                  15
SEQ ID NO: 3

Pro Ser Val Ser Gln Thr Ser Lys Leu Thr Arg Ala Glu Thr Val Phe Pro Asp Val
1               5                   10                  15

In an alternative embodiment, the linker is 90% identical to one of SEQ ID NO: 2 and SEQ ID NO: 3. In another embodiment, it is 80% identical to one of SEQ ID NO: 2 and SEQ ID NO: 3. In still another embodiment, it is 70% identical to one of SEQ ID NO: 2 and SEQ ID NO: 3. In still another embodiment, it is 60% identical to one of SEQ ID NO: 2 and SEQ ID NO: 3. In a further embodiment, it is 50% identical to one of SEQ ID NO: 2 and SEQ ID NO: 3.

Preferably, the fusion protein of the invention has the sequence as set forth in SEQ ID NO: 1 (see Figure 1). Alternatively, the sequence of the fusion protein has at least 70% identity to the sequence set forth in SEQ ID NO: 1. The sequence of the fusion protein may have at least 75% identity to the sequence set forth in SEQ ID NO: 1. The sequence of the fusion protein may have at least 80% percent identity to the sequence set forth in SEQ ID NO: 1. The sequence of the fusion protein may have at least 85% percent identity to the sequence set forth in SEQ ID NO: 1. The sequence of the fusion protein may have at least 90% percent identity to the sequence set forth in SEQ ID NO: 1. The sequence of the fusion protein may have at least 95% percent identity to the sequence set forth in SEQ ID NO: 1. The sequence of the fusion protein may have at least 98% percent identity to the sequence set forth in SEQ ID NO: 1. The sequence of the fusion protein may have at least 99% percent identity to the sequence set forth in SEQ ID NO: 1.

For the purposes of the invention, the subject to be administered the fusion protein is is a human that suffers from hemophilia B.

For any of the embodiments of the invention, the fusion protein is preferably provided for administration at a concentration of about 100 to 400 IU/ml, preferably about 100, 200 or 400 IU/ml. The fusion protein may also be provided for administration at concentrations of 600 IU/ml or 1200 IU/ml.

### Detailed description of the invention

"Prophylactic treatment", as used herein, means administering a Factor IX fusion protein in multiple doses to a subject over a course of time to increase the level of Factor IX activity in a subject's plasma. Preferably, the increased level is sufficient to decrease the incidence of spontaneous bleeding or to prevent bleeding in the event of an unforeseen injury. Prophylactic treatment decreases or prevents bleeding episodes, for example, those described under on-demand treatment.

"Dosing interval", as used herein, means the amount of time that elapses between multiple doses being administered to a subject.

"Median dose", as used herein, means half of the study subjects used higher than that dose and half of the study subjects used lower than that dose. "Mean dose" means an average dose (is computed by adding up all the doses and dividing by the total number of the doses).

"Annualized bleeding rate" means the calculated total number of bleeding events of a subject over the course of one year (number of bleeding episodes) / (observed treatment period of interest)*365.25). "Annualized spontaneous bleeding rate" means the calculated number of spontaneous bleeding events of a subject over the course of one year (number of spontaneous bleeding episodes) / (observed treatment period of interest)*365.25).

"Maintaining the plasma level of FIX at a trough of at least about" a certain percentage means, that the FIX biological activity in plasma will not fall below said percentage level during a certain dosing regimen of a patient in need of FIX, wherein 100% of said FIX biological activity correspond to 1 U/ml which is the FIX activity concentration in normal human plasma, and wherein the FIX biological activity preferably is determined using a validated one-stage clotting method as described in the Examples.

The "trough" is the lowest level of said FIX biological activity throughout the dosing regimen during the treatment of a patient in need of FIX. Due to patient inter-variability, the trough level generally refers to median values, which means that half of the study subjects had a higher trough level and half of the study subjects had a lower trough level. Though using the median value is more common, the trough level from the PK data could also be calculated as a mean value, which has been determined by adding up the values for all patients and dividing by the number of patients.

"Baseline" means the FIX activity level in a given patient preferentially expressed in IU/dL or in % of the FIX activity in a healthy person which is defined to be 100 IU/dL or 100%. In severe hemophilia B the baseline level of a given patient is very low to zero or almost zero, whereas in mild hemophilia the patient's baseline may be higher such as above 1%, above 2% or above 3% or above 4% or above 5% of the FIX activity concentration in a healthy person. When the fusion protein comprising i) a human Factor IX (FIX) portion, and ii) human albumin is administered according to the present invention first the FIX activity concentration sharply increases and is the slowly cleared i.e. it is returning to the individual baseline level. Especially in severe hemophilia B care has to be taken that the FIX activity concentration does not fall below a minimal level in order to prevent bleeding. This minimal level is called the trough level. In a severe hemophilia B patient when the baseline is practically zero a trough level of 1% above baseline means a FIX activity concentration of about 1% of the FIX activity concentration in a healthy person. In a mild hemophilia B patient having a baseline level of 3% FIX activity of the FIX activity concentration in a healthy person a trough level of 1% above baseline means a FIX activity concentration of about 4% of the FIX activity concentration in a healthy person.

In the invention, the dosing interval is once every 21 days

The therapeutic doses that may be used in the methods of the invention are about 90-110 IU/kg, about 90-95 IU/kg, about 95-100 IU/kg, about 100-105 IU/kg, and about 105-110 IU/kg.

The invention contemplates doses of, about 90-110 IU/kg, about 90-105 IU/kg, about 90-100 IU/kg, about 90-95 IU/kg, about 95-110 IU/kg, about 95-105 IU/kg, about 95-100 IU/kg, about 100-110 IU/kg, about 100-105 IU/kg, about 105-110 IU/kg, about 90 IU/kg, about 95 IU/kg, about 100 IU/kg, about 105 IU/kg or about 110 IU/kg. In a highly preferable embodiment, the dose is about 100 IU/kg, most preferably 100 IU/kg.

Preferred doses and dosing intervals are: about 90-110 IU/kg once every 3 weeks, about 90-105 IU/kg once every 3 weeks, about 90-100 IU/kg once every 3 weeks, about 90-95 IU/kg once every 3 weeks, about 95-110 IU/kg once every 3 weeks, about 95-105 IU/kg once every 3 weeks, about 95-100 IU/kg once every 3 weeks, about 100-110 IU/kg once every 3 weeks, about 100-105 IU/kg once every 3 weeks, about 105-110 IU/kg once every 3 weeks, about 90 IU/kg once every 3 weeks, about 95 IU/kg once every 3 weeks, about 100 IU/kg once every 3 weeks, about 105 IU/kg once every 3 weeks, and about 110 IU/kg once every 3 weeks. The most preferred dose and dosing interval is 100 IU/kg once every 3 weeks (21 days) The prior art has not disclosed the clinical efficacy of a three week dosing interval.

Encompassed in this invention is an embodiment, where the median plasma level of FIX activity maintains a trough of at least about 5% above baseline for the entire dosing interval of 21 days after administration of a 100 IU/kg rIX-FP dose. Also encompassed in this invention is an embodiment, where a 100 IU/kg rIX-FP dose is applied every 21 days, the median plasma level of FIX activity maintains trough of at least about 4% above baseline for the entire dosing interval. Alternatively, the plasma level of FIX activity could be calculated as a mean.

Also encompassed in this invention is an embodiment, where the median plasma level of FIX activity maintains a trough of at least about 14%, 3% or 2% above baseline for the entire dosing interval of 21 days after administration of a 100 IU/kg rIX-FP dose. Also encompassed in this invention is an embodiment, where a 100 IU/kg rIX-FP dose is applied every 21 days, the median plasma level of FIX activity maintains trough of at least about 1% above baseline for the entire dosing interval. Alternatively, the plasma level of FIX activity could be calculated as a mean.

In particular, the present invention provides prophylactic dosing regimens for a fusion protein comprising FIX and human albumin, wherein the Factor IX (FIX) portion is connected to the human albumin via a peptide linker cleavable by proteases involved in coagulation or activated by coagulation enzymes.

The linker allows cleavage before activation of FIX. The rFIX-FP fusion protein shows an increased half-life of FIX due to the human albumin but since cleavage occurs before the bleeding event, the half-life of activated FIX is reduced before activation.

Proteolytic cleavage in a coagulation-related mode, in the sense of the invention, is any proteolytic cleavage that occurs as a consequence of the activation of at least one coagulation factor or coagulation cofactor. The coagulation factor is activated almost in parallel to the proteolytic cleavage of the linker peptide (see Figure 1). Activation may occur, for example by proteolytic cleavage of the coagulation factor or by binding to a cofactor. The albumin increases the half-life of FIX in the blood until a bleeding event occurs, the bleeding event simultaneously activates FIX and cleaves it from albumin. The cleavage liberates the polypeptide from any activity-compromising steric hindrance caused by the albumin and thereby allows the generation of fusion proteins, which retain a high molar specific activity of the FIX. The cleaved FIX is then rapidly cleared from the blood due to the loss of albumin. Such fusion proteins exhibit improved half-life and molar specific activities that are increased in comparison to their non-cleavable counterparts. Thus, less rIX-FP is needed to provide a therapeutic effect compared to non-cleavable fusion proteins comprising rIX.

Preferred fusion proteins according to the invention are those that have a molar specific activity, in particular a molar specific coagulation-related activity of the therapeutic fusion protein that is increased at least 25% compared to that of the therapeutic fusion protein linked by a non-cleavable linker having the amino acid sequence GGGGGGV (SEQ ID NO: 4) in at least one coagulation-related assay. More preferred are fusion proteins in which the molar specific activity is increased by at least 50%, even more preferred those in which the molar specific activity is increased by at least 100%, in at least one of the different coagulation-related assays available.

In a further embodiment, the linker peptide comprises cleavage sites for more than one protease. This can be achieved either by a linker peptide that can be cleaved at the same position by different proteases or by a linker peptide that provides two or more different cleavage sites. There may be advantageous circumstances where the therapeutic fusion protein must be activated by proteolytic cleavage to achieve enzymatic activity and where different proteases may contribute to this activation step. Activation of FIX can either be achieved by FXla or by FVlla/Tissue Factor (TF). In a preferred embodiment, the linker is cleavable by FIXa and/or by FVlla/Tissue Factor (TF).

rIX-FP (recombinant human FIX-human albumin fusion protein) having the sequence set forth in SEQ ID NO: 1, has prolonged circulation in plasma as shown by the 5.3-fold longer half-life (t_{1/2}), the 7-fold reduced CL, and the 7-fold greater AUC compared to rFIX (e.g., Benefix^{®}). Moreover, when comparing pharmacokinetic parameters of rIX-FP to the corresponding pharmacokinetic data of FIX-Fc in the Alprolix^{™} FDA prescribing information, rIX-FP has an about 3.8-fold higher AUC0-inf, an about 3.7-fold reduced CL/BW, an about 26% higher incremental recovery, an about 3.2-fold lower volume of distribution, and an increased mean residence time of the drug by about 19%, when compared to rIX-Fc (ALPROLIX^{™}).

Prophylaxis is the treatment by intravenous injection of factor concentrate in order to prevent anticipated bleeding. Prophylaxis was conceived from the observation that moderate hemophilia patients with clotting factor level >1 IU/dl (>1%) seldom experience spontaneous bleeding and have much better preservation of joint function. Therefore, prophylaxis with FIX activity maintained above 1% to prevent bleeding and joint destruction should be the goal of therapy to preserve normal musculoskeletal function (GUIDELINES FOR THE MANAGEMENT OF HEMOPHILIA, 2nd edition, Prepared by the Treatment Guidelines Working Group, on behalf of the World Federation of Hemophilia (WFH)). A single dose of rIX-FP is capable to maintain FIX activity above 1% for 14 days and beyond.

The decreased frequency of injections reduces the risk of infections, discomfort for patients, and the number of required visits to a medical professional. These advantages will positively affect patient compliance and thus the effectiveness of prophylactic therapy for hemophilia.

### Human FIX

Human FIX, one member of the group of vitamin K-dependent polypeptides, is a single-chain glycoprotein with a molecular weight of 57 kDa, which is secreted by liver cells into the blood stream as an inactive zymogen of 415 amino acids. It contains 12 γ-carboxy-glutamic acid residues localized in the N-terminal Gla-domain of the polypeptide. The Gla residues require vitamin K for their biosynthesis. Following the Gla domain there are two epidermal growth factor domains, an activation peptide, and a trypsin-type serine protease domain. Further posttranslational modifications of FIX encompass hydroxylation (Asp 64), N- (Asn157 and Asn167) as well as O-type glycosylation (Ser53, Ser61, Thr159, Thr169, and Thr172), sulfation (Tyr155), and phosphorylation (Ser158).

FIX is converted to its active form, Factor IXa, by proteolysis of the activation peptide at Arg145-Ala146 and Arg180-Val181 leading to the formation of two polypeptide chains, an N-terminal light chain (18 kDa) and a C-terminal heavy chain (28 kDa), which are held together by one disulfide bridge. Activation cleavage of Factor IX can be achieved in vitro e.g., by Factor Xla or Factor Vlla/TF. Factor IX is present in human plasma in a concentration of 5-10 µg/ml. Terminal plasma half-life of Factor IX in humans was found to be about 15 to 18 hours (White GC et al. 1997. Recombinant Factor IX. Thromb Haemost. 78: 261-265; Ewenstein BM et al. 2002. Pharmacokinetic analysis of plasma-derived and recombinant F IX concentrates in previously treated patients with moderate or severe hemophilia B. Transfusion 42:190-197).

**Half-life enhancing albumin** The terms "human serum albumin" (HSA) and "human albumin" (HA) are used interchangeably in this application. The terms "albumin" and "serum albumin" are broader, and encompass human serum albumin (and fragments and variants thereof) as well as albumin from other species (and fragments and variants thereof).

As used herein, "albumin" refers collectively to albumin polypeptide or amino acid sequence, or an albumin fragment or variant having one or more functional activities (e.g., biological activities) of albumin. In particular, "albumin" refers to human albumin or fragments thereof, especially the mature form of human albumin. For example, albumin can have a sequence or variant thereof, as described in US2008260755A1, The albumin portion of the albumin fusion proteins may comprise the full length of the HA sequence, or may include one or more fragments thereof that are capable of stabilizing or prolonging the therapeutic activity. Such fragments may be of 10 or more amino acids in length or may include about 15, 20, 25, 30, 50, or more contiguous amino acids from the HA sequence or may include part or all of specific domains of HA.

The human albumin portion of the fusion proteins of the invention may be a variant of normal HA, either natural or artificial. The therapeutic polypeptide portion of the fusion proteins of the invention may also be variants of the corresponding therapeutic polypeptides as described herein. The term "variants" includes insertions, deletions, and substitutions, either conservative or non-conservative, either natural or artificial, where such changes do not substantially alter the active site, or active domain that confers the therapeutic activities of the therapeutic polypeptides, as described in US2008260755A1,
The invention specifically relates to fusion proteins comprising linking human factor IX to the N-terminus of human albumin such that an intervening cleavable peptide linker is introduced between the therapeutic polypeptide and the human albumin such that the fusion protein formed has an increased in vivo half-life compared to the coagulation factor which has not been linked to human albumin and that the fusion protein has an at least 25% higher molar specific activity compared to the corresponding fusion protein with non-cleavable linker in at least one of the different coagulation-related assays available.

"Factor IX" within the above definition includes polypeptides that have the natural amino acid sequence including any natural polymorphisms. It also includes polypeptides with a slightly modified amino acid sequence, for instance, a modified N-terminal or C-terminal end including terminal amino acid deletions or additions, as long as those polypeptides substantially retain the activity of the respective therapeutic polypeptide. Variants included differ in one or more amino acid residues from the wild type sequence. Examples of such differences may include truncation of the N- and/or C-terminus by one or more amino acid residues (e.g. preferably 1 to 30 amino acid residues), or addition of one or more extra residues at the N- and/or C-terminus, as well as conservative amino acid substitutions, i.e., substitutions performed within groups of amino acids with similar characteristics, e.g. (1) small amino acids, (2) acidic amino acids, (3) polar amino acids, (4) basic amino acids, (5) hydrophobic amino acids, and (6) aromatic amino acids. Examples of such conservative substitutions are shown in the following table.

**Table 1: Conservative substitutions of amino acids**

| | | | | |
|---|---|---|---|---|
| (1) | Alanine | Glycine | | |
| (2) | Aspartic acid | Glutamic acid | | |
| (3a) | Asparagine | Glutamine | | |
| (3b) | Serine | Threonine | | |
| (4) | Arginine | Histidine | Lysine | |
| (5) | Isoleucine | Leucine | Methionine | Valine |
| (6) | Phenylalanine | Tyrosine | Tryptophan | |

The in vivo half-life of the fusion proteins of the invention, in general determined as terminal half-life or β-half-life, is usually at least about 25%, preferably at least about 50%, and more preferably more than 100% higher than the in vivo half-life of the non-fused polypeptide.

The fusion proteins of the present invention have at least a 25%, preferably at least a 50%, more preferably an at least 100% increased molar specific activity compared to the corresponding fusion proteins without cleavable linkers.

The molar specific activity (or molar specific coagulation-related activity as considered here in particular) in this regard is defined as the activity expressed per mole (or e.g. nmole) of the therapeutic polypeptide or therapeutic fusion protein of interest. Calculation of the molar specific activity allows a direct comparison of the activity of the different constructs which are not affected by the different molecular weights or optical densities of the polypeptides studied. The molar specific activity may be calculated as exemplified in Table 2 below for FIX and a FIX-FP fusion protein.

**Table 2: Calculation of molar specific activity as shown for a purified FIX-HSA fusion protein**

| Product | OD_{(280nm, 1%)} | MW | Activity/Vol/OD₂₈₀ (IU/L/OD₂₈₀) | Molar optical density (OD₍₂₈₀₎ at 1 mol/L) | Calculation of molar specific activity (IU/mol) |
|---|---|---|---|---|---|
| FIX | 13.3 ¹) | 57 000 | determined for product | 75810 (= MW × OD_{(280, 1%)}/10) | =(Activity/Vol/OD₂₈₀) × (OD₂₈₀ at 1 mol/L) |
| Albumin | 5.7²) | 66 300 | | 37791 (= MW × OD₍₂₈₀, _{1%)}/10) | |
| FIX-FP | | | determined for product | 113601 (= sum of molar optical density of FIX and albumin) | = (Activity/Vol/OD₂₈₀) × (OD₂₈₀ at 1mol/L) |

| | | | | | |
|---|---|---|---|---|---|
| 1) R.G. Di Scipio et al., Biochem. 16:6698-706 (1977) 2) C. Chaudhury et al. J. Exp. Med. 197(3):315-322 (2003) | | | | | |

In order to determine a molar specific coagulation-related activity, any assay may be used that determines enzymatic or cofactor activities that are relevant to the coagulation process.

Therefore "coagulation-related assays" in the sense of the invention is any assay which determines enzymatic or cofactor activities that are of relevance in the coagulation process or that is able to determine that either the intrinsic or the extrinsic coagulation cascade has been activated. The "coagulation-related" assay thus may be direct coagulation assays like aPTT, PT, or the thrombin generation assays. However, other assays like, e.g., chromogenic assays applied for specific coagulation factors are also included. Examples for such assays or corresponding reagents are Pathromtin^{®} SL (aPTT assay, Dade Behring) or Thromborel^{®} S (Prothrombin time assay, Dade Behring) with corresponding coagulation factor deficient plasma (Dade Behring), Thrombin generation assay kits (Technoclone, Thrombinoscope) using e.g. coagulation factor deficient plasma, chromogenic assays like Biophen Factor IX (Hyphen BioMed), Staclot^{®} FVlla-rTF (Roche Diagnostics GmbH), Coatest^{®} Factor VIII:C/4 (Chromogenix), or others.

For purposes of this invention, an increase in any one of the above assays or an equivalent coagulation-related assay is considered to show an increase in molar specific activity. For example, a 25% increase refers to a 25% increase in any of the above or an equivalent assay.

For FIX, aPTT assays are often used for determination of coagulation activity. However, other coagulation-related assays or assay principles may be applied to determine molar specific activity for FIX.

Although it is desirable to have a high in vivo recovery and a long half-life for a non-activated coagulation factor, it is advantageous to limit the half-life of a coagulation factor after its activation or the activation of its co-factor in order to avoid a prothrombotic risk. Therefore, after the coagulation process has been initiated, the half-life of the active coagulation factor should again be reduced. This can either be achieved by enhancing inactivation in a coagulation-related mode or by elimination of the coagulation factor.

Inactivation according to the present invention means the decrease of activity of the therapeutic polypeptide which can be caused, for example, by a complex formation of a coagulation factor and an inhibitor of the corresponding coagulation factor or by further proteolytic cleavage as known, e.g., in the case of FVIII and FV.

The inactivation rate of an activated therapeutic fusion protein is defined as the rate the activity is declining, e.g., by reaction with inhibitors or by proteolytic inactivation. The inactivation rate may be measured by following the molar specific activity of the activated coagulation factor over time in the presence of physiologic amounts of inhibitors of this coagulation factor.

Alternatively, the inactivation rate may be determined after administration of the activated product to an animal followed by testing of plasma samples at an appropriate time frame using activity and antigen assays.

The elimination rate of an activated therapeutic fusion protein is defined as the rate the polypeptide is eliminated from the circulation of humans or animals. The elimination rate may be determined by measuring the pharmacokinetics of the activated, therapeutic fusion protein after intravenous administration. Using an antigen assay, the elimination by direct removal from the circulation can be determined. Using an activity assay in addition, a specific removal and inactivation rate may be determined.

According to this invention, the FIX is coupled to the N-terminus of human albumin via a linker cleavable by proteases involved in coagulation or activated by coagulation enzymes. The linker should be non-immunogenic and should be flexible enough to allow cleavage by proteases.

The cleavable linker preferably comprises a sequence derived from
a) the therapeutic polypeptide to be administered itself if it contains proteolytic cleavage sites that are proteolytically cleaved during activation of the therapeutic polypeptide,
b) a substrate polypeptide of this therapeutic polypeptide, or
c) a substrate polypeptide cleaved by a protease which is activated or formed by the direct or indirect involvement of the therapeutic polypeptide.

The linker region in a more preferred embodiment comprises a sequence of the therapeutic polypeptide to be applied, which should result in a decreased risk of neoantigenic properties of the expressed fusion protein.

The linker sequence is either derived from the sequences of the activation regions of FIX, from the cleavage region of any substrate of FIX like FX or FVII or from the cleavage region of any substrate polypeptide that is cleaved by a protease in whose activation FIXa is involved.

In a highly preferred embodiment the linker peptide is derived from FIX itself. In another preferred embodiment the linker peptide is derived from FX or FVII. In another preferred embodiment the linker sequence comprises two cleavage sequences that can be cleaved by FXla or FVIIa/TF, two physiologically relevant activators of FIX.

Variants and fragments of the described linkers are also encompassed in the present invention as long as the linker can still be cleaved by the protease or the proteases that cleave the linkers. The term "variants" includes insertions, deletions and substitutions, either conservative or non-conservative.

### Pharmaceutical Compositions and Modes of Administration

The fusion proteins of the invention can be incorporated into pharmaceutical compositions suitable for administration. Such compositions typically comprise the protein and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical); transmucosal, and rectal administration. Solutions or suspensions used for parenteral, intradermal, or subcutaneous, application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases; such as hydrochloric acid of sodium hydroxide. The parenteral preparation can be enclosed in ampoules, disposable syringes or multiple dose vials made of glass of plastic. Administration as an intravenous injection is the claimed route of administration.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL (BASF, Parsippany, N.J.) or phosphate buffered saline (PBS). In all cases, the composition must be sterile and should be fluid to the extent that easy syringe ability exists. It must be stable under the conditions of manufacture and storage and must be preserved against the contaminating action of microorganisms such as bacteria and fungi. The carrier can be a solvent or dispersion medium containing, for examples, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., rlX-FP) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

The formulations for injection can contain the Factor IX (FIX) fusion protein (e.g., rFIX-FP) in a therapeutically effective amount, which amount may be determined by the skilled person. In particular, the Factor IX (FIX) fusion protein (e.g., rFIX-FP) may administered at a concentration of about 100 to 400 IU/ml. For example, the fusion protein may be provided for administration at a concentration of about 100, 200 or 400 IU/ml. The fusion protein may be provided for administration at higher concentrations such as 600 IU/ml and 1200 IU/ml.

It is especially advantageous to formulate pharmaceutical compositions, such as compositions for injection, in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms are dictated by and directly dependent on the unique characteristics of the active compound and the particular therapeutic effect to be achieved.

The pharmaceutical compositions can be included in a container, pack, or dispenser together with instructions for administration.

### Description of the Figure

**Figure 1****:** Amino Acid sequence of mature rIX-FP (SEQ ID NO: 1). FIX is shown in aa 1-416, the linker sequence is in bold and underlined (aa 416-433) and the albumin sequence is shown in aa 434-1018.

Having now described the present invention in detail, the same will be more clearly understood by reference to the following examples, which are included herewith for purposes of illustration only and are not intended to be limiting of the invention.

### Examples

### Example 1 - A Study of a 21-day Prophylactic Dosing Regimen with Recombinant Coagulation Factor IX Albumin Fusion Protein (rIX-FP) in Subjects with Hemophilia B

The present study aimed to evaluate the efficacy of a 21-day prophylactic regimen for administering rIX-FP (recombinant human FIX-human albumin fusion protein) for preventing bleeding episodes, as well as assessing the safety and pharmacokinetics (PK) of this dosing interval.

A 14-day prophylactic regimen with a rIX-FP 50-75 IU/kg was effective in preventing bleeding episodes in hemophilic B patients resulting in an ABR of 2.13 (median 0.92, Q1:Q3: 0, 3.19) and an AsBR value of 1.19 (median 0, Q1:Q3: 0, 1.58) (n=48 with an average duration of 490 days). In this study, it was investigated whether a dosing interval of 21 days with 100 IU/kg rlX-FP would be effective in preventing bleeding episodes in these hemophilic patients.

### Patients

All patients had congenital Factor IX (FIX) deficiency (hemophilia B). Eleven patients who were previously undergoing the 14-day regimen of rIX-FP (48 patients) were switched to a 21-day regimen. Subjects received intravenously 100 IU/kg rIX-FP prophylactic treatment every 21 days. Under the 14-day regimen, these patients had an ABR value of 0.47 (median: 0, Q1:Q3, 0,1) and an AsBR value of 0.18 (median: 0, Q1:Q3: 0,0) and had been treated using the 14 day regimen for 295 days. All bleeding events which occurred during the active treatment period of the study were treated with rIX-FP. All subjects self-administered rIX-FP treatment for both routine prophylaxis and the treatment of bleeding events, treatments and bleeding events were recorded by subjects in an electronic diary.

### Trial objectives and endpoints

The primary objective of the study was to evaluate the clinical response of the 21-day routine prophylaxis administration regimen with rIX-FP with respect to the prevention of bleeding treated with rIX-FP.

### Analytical methods

FIX activity was measured using a validated one-stage clotting method. Briefly, the test samples were mixed with equal amounts of FIX depleted plasma and tested by *in vitro* determination of activated partial thromboplastin time (aPTT) using Pathromtin SL (Siemens Healthcare Diagnostics, Marburg) as activator reagent, rIX-FP activity determination was performed using the Behring Coagulation System (BCS). The results were interpreted using a reference curve, which was prepared from standard human plasma (SHPL) calibrated by the manufacturer against WHO standard (International Blood Coagulation Factors II,VII, IX, X, Human, Plasma) for FIX, and the results are reported in percent of norm or International Units.

Inhibitors were titrated by the Bethesda method according to the Nijmegen modification, a coagulation assay based on *in vitro* determination of aPTT in human citrated plasma. A result ≥ 0.6 Bethesda Units (BU) was defined as a positive result.

The analyses of FIX activity, FIX antigen, inhibitors and antibodies against rIX-FP were performed in the central laboratory at CSL Behring, Marburg, Germany.

### Efficacy

The numbers of infusions to achieve hemostasis were tabulated to determine the efficacy of rIX-FP for the treatment of bleeding events. In addition, the investigators rated the efficacy of the treatment of bleeding events on a four-point scale, which took into account both the number of infusions taken by the subject as well as the subject-reported pain relief after treatment.

The annualized spontaneous bleeding rate for 21-day regimen was derived by the number of spontaneous bleedings required treatment during 21-day treatment period, with at least 12 weeks of treatment completed.

### Drug product

rIX-FP is a single chain glycoprotein with a molecular weight of approximately 125,000 Da, synthesized in CHO cells. The manufacturing and formulation do not include the addition of excipients from animal or human origin (Metzner HJ, Weimer T, Kronthaler U, et al. Genetic fusion to albumin improves the pharmacokinetic properties of Factor IX. Thrombosis and Haemostasis 2009; 102: 634-44).

rIX-FP is a highly purified recombinant fusion protein linking recombinant human coagulation FIX with recombinant human albumin by a short, cleavable linker derived from an endogenous FIX sequence involved in FIX activation. The linker is cleaved from the fusion protein by the same enzymes, e.g., coagulation Factor Xla or Factor Vila/Tissue Factor, which activate FIX during the process of blood coagulation, removing the albumin moiety. rIX-FP was supplied as a lyophilized sterile formulation intended for IV injection in single-use vials of 500 and 1000 IU/vial, and was reconstituted with 2.5 mL sterile water for injection.

### Results

### Dose

All subjects were given an intravenous 100 IU/kg prophylaxis dose of rIX-FP every 21 days for an average duration of 429 days (see Table 3). This is similar to the length of time for which rIX-FP was administrated at a dosing interval of 7, 10 and 14 days. Also shown in Table 3 is the average duration of rIX-FP administration for the 7-day, 10-day and 14-day regimen studies.

**Table 3: Duration (Days) of Treatment Period for Bleeding Episodes by Regimen**

| **Statistics** | **7-Day Regimen** | **10-Day/3x-Per Month Regimen** | **14-Day Regimen** | **21-Day Regimen** |
|---|---|---|---|---|
| n | 44 | 24 | 48 | 11 |
| Mean (STD) | 465.7 (255.22) | 452.1 (312.95) | 490.2 (246.40) | 429.3 (216.91) |
| Median | 459.0 | 449.0 | 518.5 | 491.0 |
| Min, Max | 42, 890 | 11,875 | 86, 869 | 64, 693 |
| Q1, Q3 | 224.5, 700.0 | 174.5, 756.5 | 252.5, 725.0 | 146.0, 586.0 |
| Sum | 20489 | 10850 | 23531 | 4722 |

### Efficacy

The 21-day protocol resulted in very low mean AsBR and mean ABR values. The ABR was 1.1 (median: 0, Q1:Q3: 0, 2.1) and AsBR was 0.53 (median: 0, Q1:Q3: 0,0) (one subject switched from 14-day to 21 days had a less than 8 week duration, and the bleeding rate was not used for the calculation). The results are shown in Table 4.

**Table 4: AsBR and ABR by Regimen**

| | **Statistics** | **7-Day Regimen** | **10-Day/ 3x-Per Month Regimen** | **14-Day Regimen** | **21-Day Regimen** |
|---|---|---|---|---|---|
| **Total Annualized Bleeding Rate** | n | 42 | 21 | 48 | 10 |
| | Mean (STD) | 2.90 (3.009) | 2.45 (2.801) | 2.13 (2.887) | 1.10 (1.628) |
| | Median | 2.04 | 1.25 | 0.92 | 0.00 |
| | Min, Max | 0.0, 11.4 | 0.0, 10.4 | 0.0, 12.1 | 0.0, 4.7 |
| | Q1, Q3 | 0.00, 4.66 | 0.43, 4.01 | 0.00, 3.19 | 0.00,2.11 |
| **Spontaneous Annualized Bleeding Rate** | n | 42 | 21 | 48 | 10 |
| | Mean (STD) | 1.31 (2.360) | 0.87 (1.865) | 1.19 (2.167) | 0.53 (1.491) |
| | Median | 0.00 | 0.00 | 0.00 | 0.00 |
| | Min, Max | 0.0, 11.4 | 0.0, 7.6 | 0.0, 10.4 | 0.0, 4.7 |
| | Q1, Q3 | 0.00, 1.57 | 0.00, 0.83 | 0.00, 1.58 | 0.00, 0.00 |

The variation of bleeding rates between patients receiving rIX-FP every 21 days is shown in Table 5. Only three patients had an ABR above 2 and only one patient had an AsBR above 1. Apart from slightly higher bleeding rates for subject 0020, the results were highly consistent demonstrating extremely low bleeding rates for patients on the 21-day administration protocol.

**Table 5: Individual bleeding rates between patients receiving rIX-FP every 21 days**

| **Subject ID** | **Regimen** | **Age Group** | **Duration on Study (Days)** | **Total Treatment Duration (days)** | **ABR** | **AsBR** |
|---|---|---|---|---|---|---|
| 1 | 21-Day Regimen | >=12-65 yrs | 772 | 385 | 4.7 | 4.7 |
| 2 | 21-Day Regimen | >=12-65 yrs | 784 | 487 | 0.0 | 0.0 |
| 3 | 21-Day Regimen | >=12-65 yrs | 793 | 491 | 0.0 | 0.0 |
| 4 | 21-Day Regimen | >=12-65 yrs | 786 | 526 | 0.0 | 0.0 |
| 5 | 21-Day Regimen | >=12-65 yrs | 860 | 586 | 0.0 | 0.0 |
| 6 | 21-Day Regimen | >=12-65 yrs | 759 | 146 | 2.5 | |
| 7 | 21-Day Regimen | >=12-65 yrs | 648 | 64 | | |
| 8 | 21-Day Regimen | >=12-65 yrs | 886 | 693 | 2.1 | 0.0 |
| 9 | 21-Day Regimen | >=12-65 yrs | 874 | 547 | 0.0 | 0.0 |
| 10 | 21-Day Regimen | >=12-65 yrs | 840 | 652 | 1.7 | 0.6 |
| 11 | 21-Day Regimen | >=12-65 yrs | 632 | 145 | 0.0 | 0.0 |

### Updated Results based on a datacut at the end of March 2017

83 patients had entered the study overall.

**Table 6: Demographics**

| | ≥12 years n=59 | <12 years n=24 | Total N=83 |
|---|---|---|---|
| Age (years), mean (range) | 36.0 (13-63) | 7.0 (2-11) | 27.7 (2-63) |
| <18 years, n (%) | 5 (8.5) | 24 (100) | 29 (34.9) |
| ≥18 years, n (%) | 54 (91.5) | 0 | 54 (65.1) |

| Race, n (%) | | | |
|---|---|---|---|
| White | 45 (76.3) | 23 (95.8) | 68 (81.8) |
| Asian | 12 (20.3) | 0 | 12 (14.5) |
| Black | 2 (3.4) | 1 (4.2) | 3 (3.6) |

| Ethnicity, n (%) | | | |
|---|---|---|---|
| Hispanic | 0 | 2 (8.3) | 2 (2.4) |
| Not Hispanic | 59 (100) | 22 (91.7) | 81 (97.6) |

At the beginning of the extension study, PTPs ≥12 years were on 7-day (n=19), 10-day (n=13) or 14-day (n=27) dosing intervals. PTPs <12 years were on 7-day (n=18), 10-day (n=2) or 14-day (n=4) dosing intervals.

At the datacut, 79% of PTPs ≥12 years had dosing intervals of 10 (n=12), 14 (n=26) or 21 days (n = 9).

11 PTPs ≥18 years switched to a 21-day regimen during the study, two of which switched back to 14 days to reduce bleeding frequency.

At the datacut, 25% of the PTPs <12 years had dosing intervals of 10 (n=1) or 14 days; two patients switched back from a 14-day regimen to a 10- (n=1) or 7-day (n=1) regimen and two patients switched from a 10-day regimen to a 7-day regimen to maintain lower bleeding rates.

ABR and AsBR were calculated for each dosing regimen; the analysis includes the total number of patients who received each regimen at any timepoint during the extension study (Table 7).

**Table 7: AsBR and ABR across the different dosing regimens**

| **Regimen*** | | **7 day** | **10 day** | **14 day** | **21 day** |
|---|---|---|---|---|---|
| **≥12 years (n=59)** | | n=21 | n=17 | n=40 | n=11 |
| **AsBR** | Median (Q1,Q3) | 0.33 (0.00, 2.39) | 0.00 (0.00, 0.68) | 0.00 (0.00, 1.54) | 0.00 (0.00, 0.45) |
| | Mean (SD) | 1.38 (1.91) | 0.50 (0.90) | 1.21 (2.28) | 0.62 (1.42) |
| **ABR** | Median (Q1,Q3) | 1.43 (0.18, 4.36) | 0.71 (0.00, 3.39) | 0.73 (0.00, 2.62) | 0.00 (0.00, 2.50) |
| | Mean (SD) | 2.49 (2.632) | 1.68 (2.075) | 1.95 (2.851) | 1.18 (1.603) |
| **<12 years (n=24)** | | n=21 | n=7 | n=8 | N/A |
| **AsBR** | Median (Q1,Q3) | 0.00 (0.00, 0.59) | 0.00 (0.00, 3.06) | 0.75 (0.00, 2.86) | N/A |
| | Mean (SD) | 0.73 (1.75) | 1.52 (2.90) | 1.53 (1.86) | N/A |
| **ABR** | Median (Q1,Q3) | 2.31 (0.96, 4.21) | 2.82 (0, 6.11) | 3.11 (1.63, 5.77) | N/A |
| | Mean (SD) | 3.18 (3.259) | 3.84 (3.843) | 3.56 (2.650) | N/A |

### Discussion

This study, for the first time, supports using a 21-day dosing interval for preventing bleeding episodes since it was very effective in preventing bleeding episodes in hemophilic B patients. The bleeding rates were extremely low, especially considering the long dosing interval. Due to the inconvenience of administering rIX-FP intravenously, the longer dosing interval is advantageous and more convenient for patients. This should increase patient compliance and result in better control of bleeding events in hemophilia B patients.

## Claims

1. A fusion protein comprising
a) a human Factor IX (FIX) and
b) human albumin
for use in a method of preventing bleeding in a human suffering from hemophilia B in a prophylactic dosing regimen, wherein the FIX is connected to the N-terminus of human albumin via a peptide linker cleavable by proteases involved in coagulation or activated by coagulation enzymes, and wherein the fusion protein is to be administered to the subject at a dose of about 90-110 IU/kg for a dosing interval of once every 21 days, wherein the dose is to be administered intravenously.

2. The fusion protein for use as in any one of claim 1, wherein the dose is about 100 IU/kg.

3. The fusion protein for use as in any one of the preceding claims, wherein the annualized bleeding rate is less than 20 per year.

4. The fusion protein for use as in claim 3, wherein the annualized bleeding rate is less than 3 per year

5. The fusion protein for use as in any one of the preceding claims, wherein the annualized spontaneous bleeding rate is less than 15 per year.

6. The fusion protein for use as in claim 5, wherein the annualized spontaneous bleeding rate is less than 2 per year.

7. The fusion protein for use as any one of the preceding claims, wherein the plasma level of the FIX maintains a trough of at least about 1%, at least about 2%, at least about 3%, at least about 4%, or between 5 and 15%, above baseline for the entire dosing interval.

8. The fusion protein for use as in claim 7, wherein the plasma level of the FIX maintains a trough of at least about 2-4% above baseline for the entire dosing interval.

9. The fusion protein for use as in any one of the preceding claims, wherein the linker is cleavable by FIXa and/or by FVlla/Tissue Factor (TF).

10. The fusion protein for use as in claim 9, wherein the linker comprises a sequence selected from SEQ ID NO: 2 and SEQ ID NO: 3.

11. The fusion protein for use as in any of the preceding claims, wherein the sequence of the fusion protein has the sequence set forth in SEQ ID NO: 1.

12. The fusion protein for use as in any one of the preceding claims, wherein the fusion protein is provided for administration at a concentration of about 100 to 400 IU/ml.

## Patentansprüche

1. Fusionsprotein, umfassend
a) einen humanen Faktor IX (FIX) und
b) Humanalbumin
zur Verwendung in einem Verfahren zum Verhindern von Blutungen bei einem Menschen, der an Hämophilie B leidet, in einem prophylaktischen Dosierungsschema, wobei der FIX mit dem N-Terminus von Humanalbumin durch einen Peptidlinker verbunden ist, der durch Proteasen spaltbar ist, die an Koagulation beteiligt sind oder durch Koagulationsenzyme aktiviert werden, und wobei das Fusionsprotein dem Subjekt in einer Dosis von etwa 90-110 IE/kg für ein Dosierungsintervall einmal alle 21 Tage zu verabreichen ist, wobei die Dosis intravenös zu verabreichen ist.

2. Fusionsprotein zur Verwendung nach Anspruch 1, wobei die Dosis etwa 100 IE/kg ist.

3. Fusionsprotein zur Verwendung nach einem der vorstehenden Ansprüche, wobei die annualisierte Blutungsrate kleiner als 20 pro Jahr ist.

4. Fusionsprotein zur Verwendung nach Anspruch 3, wobei die annualisierte Blutungsrate kleiner als 3 pro Jahr ist.

5. Fusionsprotein zur Verwendung nach einem der vorstehenden Ansprüche, wobei die annualisierte spontane Blutungsrate kleiner als 15 pro Jahr ist.

6. Fusionsprotein zur Verwendung nach Anspruch 5, wobei die annualisierte spontane Blutungsrate kleiner als 2 pro Jahr ist.

7. Fusionsprotein zur Verwendung nach einem der vorstehenden Ansprüche, wobei das FIX Plasmalevel einen Tiefpunkt von mindestens etwa 1%, mindestens etwa 2%, mindestens etwa 3%, mindestens etwa 4%, oder zwischen 5 und 15%, über dem Basislevel für das gesamte Dosierungsintervall beibehält.

8. Fusionsprotein zur Verwendung nach Anspruch 7, wobei das FIX Plasmalevel einen Tiefpunkt von mindestens etwa 2-4% über dem Basislevel für das gesamte Dosierungsintervall beibehält.

9. Fusionsprotein zur Verwendung nach einem der vorstehenden Ansprüche, wobei der Linker durch FIXa und/oder durch FVIIa/Gewebefaktor (TF) spaltbar ist.

10. Fusionsprotein zur Verwendung nach Anspruch 9, wobei der Linker eine Sequenz umfasst, die ausgewählt ist aus SEQ ID NR: 2 und SEQ ID NR: 3.

11. Fusionsprotein zur Verwendung nach einem der vorstehenden Ansprüche, wobei die Sequenz des Fusionsproteins die Sequenz aufweist, die in SEQ ID NR: 1 dargelegt ist.

12. Fusionsprotein zur Verwendung nach einem der vorstehenden Ansprüche, wobei das Fusionsprotein zur Verabreichung mit einer Konzentration von etwa 100 bis 400 IE/ml bereitgestellt wird.

## Revendications

1. Protéine de fusion comprenant
a) un facteur IX humain (FIX) et
b) une albumine humaine
pour utilisation dans un procédé de prévention de saignements chez un humain atteint d'hémophilie B dans un schéma posologique prophylactique, dans laquelle le FIX est lié à l'extrémité n-terminale d'une albumine humaine via un lieur peptidique clivable par des protéases intervenant dans la coagulation ou activées par des enzymes de coagulation, et dans laquelle la protéine de fusion est à administrer au sujet à raison d'environ 90-110 Ul/kg suivant un intervalle posologie d'une prise tous les 21 jours, dans laquelle la dose est à administrer par voie intraveineuse.

2. Protéine de fusion pour utilisation selon la revendication 1, dans laquelle la dose est d'environ 100 Ul/kg.

3. Protéine de fusion pour utilisation selon l'une quelconque des revendications précédentes, dans laquelle le nombre annualisé de saignements est inférieur à 20 par an.

4. Protéine de fusion pour utilisation selon la revendication 3, dans laquelle le nombre annualisé de saignements est inférieur à 3 par an.

5. Protéine de fusion pour utilisation selon l'une quelconque des revendications précédentes, dans laquelle le nombre annualisé de saignements spontanés est inférieur à 15 par an.

6. Protéine de fusion pour utilisation selon la revendication 5, dans laquelle le nombre annualisé de saignements spontanés est inférieur à 2 par an.

7. Protéine de fusion pour utilisation selon l'une quelconque des revendications précédentes, dans laquelle la concentration plasmique du FIX maintient un minimum d'au moins environ 1%, au moins environ 2%, au moins environ 3%, au moins environ 4%, ou entre 5 et 15%, au-dessus d'un niveau de référence sur l'ensemble de l'intervalle posologique.

8. Protéine de fusion pour utilisation selon la revendication 7, dans laquelle la concentration plasmique du FIX maintient un minimum d'au moins environ 2-4% au-dessus d'un niveau de référence sur l'ensemble de l'intervalle posologique.

9. Protéine de fusion pour utilisation selon l'une quelconque des revendications précédentes, dans laquelle le lieur est clivable par FIXa et/ou par FVlla/facteur tissulaire (TF).

10. Protéine de fusion pour utilisation selon la revendication 9, dans laquelle le lieur comprend une séquence sélectionnée parmi SEQ ID NO:2 et SEQ ID NO:3.

11. Protéine de fusion pour utilisation selon l'une quelconque des revendications précédentes, dans laquelle la séquence de la protéine de fusion présente la séquence énoncée dans SEQ ID NO:1.

12. Protéine de fusion pour utilisation selon l'une quelconque des revendications précédentes, dans laquelle la protéine de fusion est fournie en vue d'une administration selon une concentration d'environ 100 à 400 UI/ml.
